# EUROPEAN PATENT APPLICATION

(11) **EP 2 712 651 A1**
(43) Date of publication of application: **02.04.2014**
(21) Application number: 12186348.4
(22) Date of filing: 27.09.2012
(51) Int. Cl.: A61M 39/10

(54) **Venting device for use in ambulatory infusion system**

(71) Applicant: F. Hoffmann-La Roche AG, 4070 Basel (CH); Roche Diagnostics GmbH, 68305 Mannheim (DE)
(72) Inventor: Kuster, Pius, 3600 Thun (CH); Moia, Franco, 4402 Frenkendorf (CH)
(74) Representative: Rentsch Partner AG

(57) **Abstract**

Disclosed is a venting device (300), including
a) a venting member (305) that is made from a hydrophobic and gas-permeable material, the venting member (305 )having an environment coupling surface and an opposed cartridge coupling surface,
b) a carrier member (315), the carrier member (315) structurally supporting the venting member (305).

The venting member is designed such that the venting device (300) and a liquid drug cartridge (100) form, in the assembled state, a compact common cartridge assembly. After inserting the cartridge assembly into a cartridge compartment of an ambulatory infusion device, the cartridge coupling surface fluidic couples, to a non liquid contacting outer surface of the movable wall, and the environment coupling surface at the same time fluidic couples to the environment.

## Description

### Technical field

The present disclosure lies in the field of ambulatory infusion systems. The disclosure in particular concerns venting devices for use in combination with drug cartridges and ambulatory infusion devices as well as alignment devices for aligning a drug cartridge and an adapter.

### Background / prior art

Ambulatory infusion devices that are designed to be carried by a patient during everyday life night and day for an extended time period are used in a number of therapies. Such devices especially form a basis for CSII (Continuous Subcutaneous Insulin Infusion), a state-of-the-art therapy of diabetes mellitus.

The insulin is typically provided in a cylindrical drug cartridge. A piston is sealing received an inner volume of the cartridge. For expelling drug out of the cartridge the piston is pushed forward from a proximal into a distal direction by a motor-driven plunger rod of the infusion device. Thereby, drug is expelled out of an outlet and into an infusion cannula, the infusion cannula being coupled to the outlet of the cartridge either directly or via intermediate fluidic components, such as tubing and, in some cases, a check-valve. The piston accordingly serves as movable wall of the cartridge such that its inner volume decreases as drug is expelled. Since ambulatory infusion devices such as insulin pumps are carried continuously during everyday life, mechanical robustness and some degree of water protection or, preferably, full water tightness are required. Therefore, the housing is often hermetically sealed during operation and only has a sealed coupling to the infusion cannula. Because drug is expelled from the cartridge which itself is arranged inside the device housing during operation, the hermetic sealing would result - without compensation measures - in a continuous decrease of the inner pressure inside the device housing over time, potentially affecting the dosing precision. Therefore, venting means are provided in form of a gas-permeable and hydrophobic membrane that may be made from Gore-Tex® or a similar material. The membrane ensures continuous pressure equalization between the inner volume of the device and the environment, while preventing water or other liquids from entering the device. In addition, the membrane ensures pressure equalization in case of varying barometric pressure, e.g. due to weather changes and/or changes in altitude.

In some current devices, the membrane is integral part of the device housing. A corresponding device is disclosed, e.g., in the EP 1732624 A1. This arrangement, however, has the drawback that the membrane is typically more and more clogged by dirt, fabric particles, and the like, over the device lifetime - typically in a range of some years. Thereby the pressure equalization is negatively effected and in some cases voided.

In alternative designs, the membrane is therefore provided in a separate adapter that serves as closure for a drug cartridge compartment of the infusion device via, e.g. a skewed or bayonet connector and additionally includes a coupler for coupling an infusion tubing to the outlet of the drug cartridge. Providing the membrane in such an adapter is an improvement in so far as the adapter is typically designed for a considerably shorter lifetime as compared to the infusion device itself, thus reducing the clogging problem. A corresponding adapter is disclosed, e.g., in the WO 20025852 A1. This arrangement, however, has the disadvantage that it can not be used in systems that do not use a separate disposable adapter. In addition, the lifetime of the adapter may still be too long to prevent clogging.

A further specific problem arises when drug cartridges are used in an ambulatory infusion device that have an outlet which is - in the isolated state - sealing closed by a self-sealing septum. For use in the infusion system, the septum is pierced by a hollow adapter cannula that fluidic couples the inner volume of the cartridge to the infusion cannula. The adapter cannula is typically part of an adapter as described above.

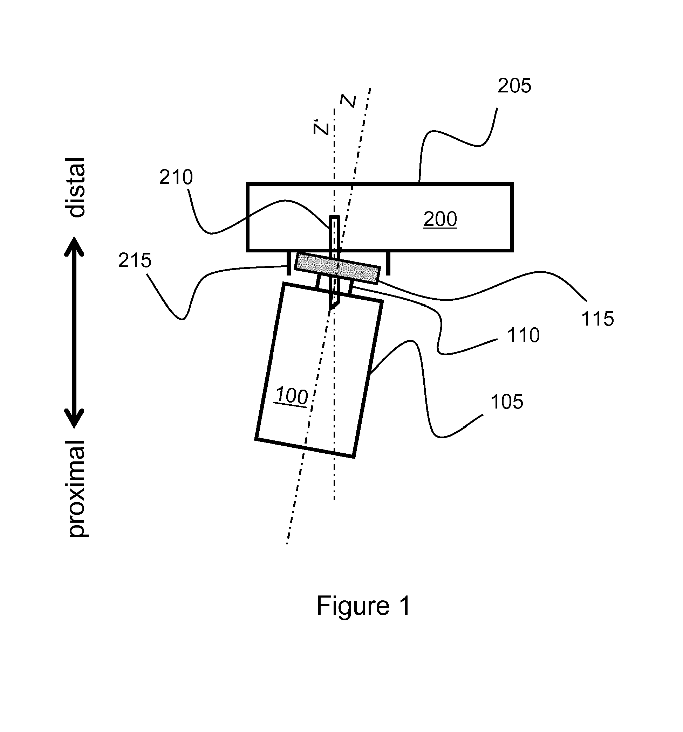

Figure 1 schematically shows a typical situation when connecting a drug cartridge with septum to an adapter. Cartridge 100 is typical cylindrical and has a (not shown) proximal piston that is sealing and sliding arranged in glass or plastic body 105, cartridge body 195 having an open proximal end. At its distal end, cartridge body 105 has a constricted neck portion 110 with cap 115 that includes a central septum (not visible). Adapter 200 has an adapter body 205 from which adapter cannula 210 projects. A (not shown) infusion tubing or coupler for an infusion tubing is part of or connected to adapter 200 for coupling to an infusion cannula. Guide 215 is provided for positioning and aligning cartridge 100 and adapter 200. Typically, Guide 215 has the form of a collar or ring that projects from a proximal bottom surface of adapter 200 and may also carry an infusion device coupler for coupling (not shown), e.g. in form of a bayonet, for coupling to the infusion device housing. Typically, guide 215 has an axial dimension of some millimetres and surrounds, i.e. has an axial overlap with, cap 115, but not cartridge body 105.

Ideally, cannula 210 pierces septum the septum perpendicular to the septum, such that the longitudinal cartridge axis Z is parallel to and preferably aligned with with longitudinal cannula axis Z'. In practice, however, substantial misalignment may be typically present, as shown (somewhat exaggerated) in Figure 1, resulting in undesired transverse forces on cannula 210 and the septum.

The possibility of significant misalignment is due to the fact that cartridge 100 is guided with respect to adapter 200 via cap 115 only which has a dimension along cartridge axis Z in a range of typically 2 mm to 3 mm, which is not sufficient to ensure axial guiding.

Consequently, the septum may leak either from beginning on or start leaking during the application time of the cartridge which is typically in a range of some days up to maybe two weeks. Guide 215 typically has an inner diameter that is somewhat larger than the outer diameter of cap 115.

To improve the situation, it would - at least theoretically - be possible to design adapter 200 with a considerably extended axial length of guide 215, resulting in guide 215 to axial overlap cartridge body 105 by an amount in a range of, e.g., 7 mm to 10 mm. In addition, guide 215 would need to be provided with tight inner tolerance.

In this context, it has to be understood, however, that adapter 200 needs to meet a considerable number of partly contradictory constraints and is further regulatory critical since it is of direct significance for ensuring reliable infusion. Therefore, the development time and effort for an adapter and the corresponding interface structure of the infusion device are considerable. In practice, it is tried to base new adapter designs on existing ones as far as ever possible, with as little and preferably no modification at all to the cartridge interface. In addition, a variety of drug cartridges exists which are typically provided by different suppliers than infusion pumps and adapters. In view of the generally complex and critical adapter design, providing different adapters that ensure appropriate cartridge guiding for a variety of existing and newly developed cartridges is practically unfeasible. As a consequence, the whole ambulatory infusion system is typically designed for use with a single type of drug cartridge only.

It is a general objective of the present disclosure to provide devices that improve the situation with respect to the above-identified problems, i.e. with respect to venting and/or cartridge alignment.

### Summary of disclosure

According to one aspect, the present disclosure is directed towards a venting device. The venting device includes a venting member, the venting member being made from a hydrophobic and gas-permeable material. The venting member has an environment coupling surface and an opposed cartridge coupling surface, thus enabling gas transfer and disabling liquid transfer across the venting member between the environment coupling surface and the cartridge coupling surface. The venting device further includes a carrier member, the carrier member structurally supporting the venting member.

The carrier member is designed for assembly to a cartridge, the cartridge storing a liquid drug in an inner volume and having a movable wall, the movable wall decreasing the inner volume upon the stored liquid drug amount decreasing.

The venting device is designed such that the venting device and the cartridge form, in the assembled state, a compact common cartridge assembly. After inserting this cartridge assembly into a cartridge compartment of an ambulatory infusion device, the cartridge coupling surface fluidic couples to a non liquid contacting outer surface of the movable wall. At the same time, the environment coupling surface fluidic couples to the environment.

According to this aspect of the present disclosure, the venting member is attached to the cartridge and inserted into the cartridge compartment together with the cartridge as common cartridge assembly, rather than being integrated into the housing of the ambulatory infusion device or into an external adapter. This kind of design increases the flexibility with respect to the adapter design and further ensures that the venting member is replaced each time together with the cartridge, while the adapter may have a longer lifetime.

By fluidic coupling the movable wall - via the venting member - to the environment it is ensured that no undesired force, resulting from an overpressure or underpressure inside the cartridge compartment - acts on the liquid drug inside the cartridge via the movable wall.

The cartridge may be a cylindrical cartridge with a cartridge body that is typically made from glass or plastic and has an open proximal end.

A piston that is typically made from plastic and/or rubber is sealing and sliding arranged in the cartridge body and serves as movable wall, thus resulting in a syringe-like design. The movable wall ensures that the liquid storing volume that is limited by an inner surface of the cartridge body and a distal inner surface of the piston is always filled with liquid drug while no or only a negligible amount of air or gas is present. This type of cartridge design is typical for current devices and generally assumed in the following, where not explicitly expressed. Alternatively, however, other cartridge designs may be used. The cartridge may, e.g. be made from one or more foil or membrane sheets and realized as fully flexible bag or pouch, or it may be semi-flexible with a rigid shell that is covered by a foil or membrane sheet. In those embodiments, the foil or membrane sheets serve as movable wall. In the context of CSII, typical maximum filling volumes of the cartridge may be in a range of, e.g., 1 ml to 4 ml, such as 1.5 ml or 3 ml.

The cartridge compartment of the ambulatory infusion device typically has a size and shape that fits to the cartridge. For a typical elongated cylindrical cartridge, the cartridge compartment typically has also cylindrical or rectangular shape, such that the cartridge fits into the cartridge compartment with some but no extensive lateral play. Typically, a plunger rod of a drive unit projects into the cartridge compartment for engaging and displacing the cartridge piston. The inner dimensions of the cartridge body, in particular the inner diameter in case of a cylindrical cartridge, must accordingly be such that a distal section of the plunger rod can be inserted into the proximal section of the cartridge body via its open proximal end.

The cartridge compartment is favourably fluidic tight sealed against other parts and components of the infusion device, in particular power supplies, drive unit components such as motors or gears, end control circuitry. The cartridge compartment may or may not be air or gas tight sealed against remaining parts of the device.

Fluidic coupling between the cartridge coupling surface of the venting member and the movable wall is favourably achieved via the inner volume of the cartridge compartment. That is, both the outer surface of the movable wall and the cartridge contacting surface of the venting member are in fluidic communication via a remaining air that is present in the cartridge compartment when the cartridge assembly is inserted, especially in form of a "gap" between cartridge body and cartridge compartment walls. A direct physical contact between movable wall and cartridge coupling surface is not required as long as fluidic coupling for free air exchange and pressure equalization is given.

The venting member may typically be made from Gore-Tex® or a comparable material. In some typical embodiments, the venting member is a membrane, the membrane having lateral dimensions that are large as compared to a membrane thickness. Due to the small thickness, the gas transfer properties of a thin membrane are particularly favourably. Alternatively, however, the venting member may have other shapes and be, e.g., in form of a small cylinder. A typical cross-sectional area of the venting member, i.e. the surface area of the environment contacting surface and the cartridge coupling surface, is in the mm²-range. In a typical embodiment - however not necessarily, the venting member is embedded in the carrier member such that the carrier member fully surrounds the venting member. The venting member may be assembled to the carrier member using techniques such as adhesive bonding, ultrasonic bonding, or force fitting.

In some embodiments, the environment coupling surface and the cartridge coupling surface have a common normal axis, the normal axis being, in the assembled state, parallel to a longitudinal cartridge axis. This type of arrangement allows a particularly compact design.

In some embodiments, the carrier member includes an elongated coupling channel, the coupling channel fluidic coupling the environment coupling surface or the cartridge coupling surface with a peripheral surface of the venting device. This type of embodiment allows an arrangement of the venting member, especially a membrane-like venting member, directly at or close to an outer surface of the carrier member, which is favourable with respect to assembly. While direct coupling is typically achieved for one of the surfaces, the other of the surfaces is fluidic coupled via the channel in the carrier member. Alternatively, however, both surfaces may be fluidic coupled via corresponding coupling channels.

In some embodiments, the carrier member surrounds, in the assembled state, at least a portion of a circumference of the cartridge.

In some embodiments, the carrier member forms a cap, the cap the being designed to be put over a distal end section of the cartridge. This type of embodiments may be realized in a number of way. Some typical cartridges, like the cartridge that is shown in above-discussed Figure 1, have an elongated cartridge body that is made from glass or plastic and has, at its distal end, a metal cap that is attached to the cartridge body via crimping or the like. The cap has a cut-out that enables access to a septum. For this type of cartridge, the cap may simultaneously serve as carrier member and the venting member may be directly integrated into a peripheral area of the cap, outside the cartridge body. For this type of design, the venting device is an integral part of the readily assembled cartridge and is provided to the user in this way. Alternatively, the carrier member may be realized as plastic cap that is put over the proximal end section either during cartridge manufacture or later on, e.g. by a patient himself immediately prior to use. While the former design may be typically applied for cartridges that are especially designed for use in an ambulatory infusion device, the latter design may also be applied where standard cartridges, that are e.g. mainly designed for use in a pen-type injection device (so-called injection pen) where venting is not required, are used.

Generally, the carrier member may be designed for assembly to the cartridge, in particular to a cartridge body, by a number of techniques. Assembly may especially carried out via at least one of snap-fit, force fit, or cramping. In embodiments of the cartridge assembly that are especially designed for use in an ambulatory infusion device, the carrier may also be part of a cap as described above or may be directly integral with the cartridge body that is e.g. made from plastic.

In some embodiments, the venting device includes an alignment structure. In such embodiments, The venting device is designed to couple to a cartridge with an elongated cartridge body that extends along a longitudinal cartridge axis and has, at its distal end, a constricted neck portion, the cartridge further including a cap with a piercable septum distal from the neck portion, the septum being perpendicular to the cartridge axis. This type of cartridge corresponds to the example of Figure 1.

This type of venting device is further designed to couple to an adapter, the adapter including an adapter cannula with a longitudinal cannula axis to pierce the septum, and an infusion device coupler to mechanically couple to and engage with a housing of an ambulatory infusion device.

This type of venting device includes a proximal cartridge engagement structure, the cartridge engagement structure being designed for axial aligned engagement with a distal end section of the cartridge body, and a distal adapter engagement structure, the adapter engagement structure being designed for axial aligned engagement with the adapter, thus enabling a coupling of the cartridge with the adapter via the venting device, wherein the adapter and the cartridge are, during the coupling, aligned by the cartridge engagement structure and the adapter engagement structure, respectively relative to each other such that the longitudinal cartridge axis and the longitudinal cannula axis form a common longitudinal axis.

According to a further aspect, the present disclosure is directed towards a cartridge assembly for use in an ambulatory infusion device, wherein the cartridge assembly includes a cartridge and a venting device according to the present disclosure. Such a cartridge assembly may be assembled by a supplier directly during production, or it may be assembled by the user as described above. The term "cartridge assembly" includes both embodiments where the venting device is integral part of the cartridge as well as embodiments where the venting device is a separate device. The cartridge assembly is designed to fit into the cartridge compartment of an ambulatory infusion device.

In some embodiments of a cartridge assembly, the venting device is arranged at a distal end section of the cartridge, the distal end section including an outlet for connecting to an infusion cannula.

In some embodiments of a cartridge assembly, the outlet includes a self-sealing, piercable septum. The cartridge of such an embodiment may, e.g., be a so called pen-type cartridge that is designed to be typically used in co combination with a hand-held pen-shape injection device. However, those cartridges may also be used in correspondingly designed ambulatory infusion systems, in which case a venting device according to the present disclosure is particularly favourable. Closing the cartridge outlet by a piercable septum is favourable in so far as septa are self-sealing, i.e., do not require further closure means, can be easily pierced by a cannula and provide a sterile barrier. Alternatively, however, the outlet may also be designed as standard Luer coupler, as bayonet, or the like.

In some embodiments of a cartridge assembly, the cartridge further includes a piston and an elongated cartridge body having an longitudinal cartridge axis, the piston being sealing arranged inside the cartridge body and being displaceable along the longitudinal cartridge axis, the piston having a liquid-contacting distal end and a non-liquid contacting proximal end, wherein the cartridge contact surface fluidic couples to the proximal end.

In some embodiments of such a cartridge assembly, the venting device is arranged at a proximal end section of the cartridge, such that the cartridge contact surface is adjacent to a proximal piston front surface and the venting device tightly covers an open proximal end of the cartridge body. In such a design, the carrier means may, e.g. be formed by a cap that is put over the proximal end section of the cartridge body, or by an , e.g. cap-like or disk-shaped, fit-in piece that is tightly fit into the proximal opening of the cartridge body, proximal of the piston. In a further alternative, the cartridge body itself serves as carrier member, and the venting member - typically in form of a membrane - is directly bonded to the proximal end section of the cartridge body, e.g., via adhesive bonding.

For this type of embodiment, the venting device is used for ensuring pressure equalization in particular prior to application during storage.

This type of embodiment is especially useful where an ambulatory infusion device is provided pre-assembled with a cartridge being already built in. Here, the whole device is typically designed for a single application. This may be the case for disposable single-use insulin pumps, as well as in other clinical therapies. The storage time after assembly and prior to use may be up to several years. During this time period, the device - including the cartridge - may experience considerable air pressure variations and may also be carried to significantly different heights above sea level. This may result in undesired over- or underpressure inside the cartridge, and potentially even undesired piston movement. In addition, the cartridge needs to be maintained sterile during this whole storage time.

This is enabled by a cartridge assembly according to this type of embodiment, since the venting member additionally serves as sterile barrier. In this way, providing and maintaining the whole device in a sterile state during the whole storage period can be avoided.

In the context of this type of embodiment, a housing of the ambulatory infusion device favourably includes a venting channel that couples the environment coupling surface of the venting device with the environment.

Sine - for this type of embodiment - the venting member is arranged between the piston and the typically present plunger rod that engages the piston during application, access to the piston must be established at the beginning of the application. The venting member may therefore be designed to be ruptured by a plunger rod of an ambulatory infusion device. This may be achieved by a strength of the venting member that does not withstand the force that is applied by the drive rod when moving in distal direction from an initial retracted position in distal direction towards the piston. The rupture may be optionally supported by a peroration of the venting member. Alternatively, the venting device may be designed to be pushed forward into the cartridge body and being permanently arranged between plunger rod and piston. In a further alternative, the venting device may flip away, e.g. via a hinge, or be removed by a device user, e.g. by a strap that is attached to or part of the venting device and projects out of the infusion device housing.

According to a still further aspect, the present disclosure is directed towards an adapter for coupling a cartridge assembly as discussed with an infusion cannula and an ambulatory infusion device, the adapter including
a) an infusion device coupler to mechanically couple to and engage with a housing of the ambulatory infusion device,
b) a drug channel to fluidic couple the inner volume of the cartridge with an infusion cannula, and
c) a venting channel for establishing, in the assembled state, a fluidic tight coupling of the environment and the environment contacting surface and the environment via the venting channel.

For a cartridge assembly as discussed above in which the cartridge and the venting assembly form a compact common unit, the cartridge assembly is inserted into the cartridge assembly as a whole. Some closure means are therefore required for establishing a fluidic coupling between environment coupling surface and environment. For this purpose, the venting channel is provided in an adapter as disclosed above. The fluid-tight connection may be realized by O-ring seals that are part of the adapter or the venting device, or sealing elements that are structural part of an adapter body or the carrier member, e.g. via providing a soft sealing member in a multi-component injection moulding process.

According to a still further aspect, the present disclosure is directed towards an adapter-cartridge unit, the adapter-cartridge-unit including a cartridge assembly as discussed above and an adapter as discussed above.

According to a still further aspect, the present disclosure is directed towards an ambulatory infusion device. The ambulatory infusion device includes a cartridge assembly with a venting device wherein the venting member is designed to be ruptured by a plunger rod of the ambulatory infusion device as described above. The ambulatory infusion device further includes a drive unit with a linearly displaceable plunger rod, the plunger rod being designed to rupture the venting member and to subsequently engage the distal piston end. The ambulatory infusion device further includes a venting channel, the venting channel fluidic coupling the environment coupling surface with the environment.

According to a still further aspect, the present disclosure is directed towards an alignment device. The alignment device is designed for coupling a liquid drug cartridge with an adapter via the alignment device.

The cartridge is a cartridge as discussed above and as shown in Figure 1. In particular, the cartridge has an elongated cartridge body that extends along a longitudinal cartridge axis and has, at its distal end, a constricted neck portion. The cartridge further includes a cap with a piercable septum distal from the neck portion, the septum being perpendicular to the cartridge axis. The adapter includes an adapter cannula with a longitudinal cannula axis to pierce the septum.

The alignment device includes a proximal cartridge engagement structure, the cartridge engagement structure being designed for axial aligned engagement with a distal end section of the cartridge body. The alignment device further includes a distal adapter engagement structure, the adapter engagement structure being designed for axial aligned engagement with the adapter.

The alignment device enables a coupling of the cartridge with the adapter. The adapter and the cartridge are, at the end and preferably also during the coupling process, aligned by the cartridge engagement structure and the adapter engagement structure, respectively relative to each other such that the longitudinal cartridge axis and the longitudinal cannula axis form a common longitudinal axis.

By providing an alignment device in accordance with the present disclosure, the cartridge is guided relative to the adapter not only along the axial height of the cap. Instead, the axial guiding length is substantially longer without requiring modification of cartridge and/or adapter. Instead, alignment device can be designed comparatively easily and fast to fit for a given cartridge and adapter. With the relative tolerances between cartridge and adapter being unaffected, the longer guiding length considerably reduces the angular play and accordingly the angular misalignment.

By aligning the longitudinal cartridge axis and the longitudinal cannula axis, i.e., by ensuring the two axis to be in coincidence, septum leakage problems as discussed above with reference to Figure 1 are avoided or at least considerably reduced. The alignment device exploits the fact that typically the cartridge is manufactured with high (rotational) symmetry about the longitudinal cartridge axis and with sufficiently tight tolerances. Via the disclosed alignment device, these properties of the cartridge are used to ensure alignment with the adapter cannula

the alignment device enables, during the coupling process, an aligned and guided relative displacement of the cartridge and the adapter towards each other along the common longitudinal axis. During the displacement, the adapter cannula comes in contact with and finally pierces the septum. This kind of embodiment ensures that correct alignment is given not only in the assembled final state, but also during the assembly process. During the coupling process, the guided and aligned engagement and coupling preferably starts at an axial distance of cartridge and adapter where the tip of the adapter cannula is still axial displaced from and does not pierce the septum.

In some embodiments, the cartridge engagement structure is designed to surround, in the assembled state, a distal section of the cartridge body and/or to project, in the assembled state, from the cap in proximal direction.In this type of embodiment, the symmetry and tight tolerances of the cartridge body are exploited for the alignment.

Alternatively or additionally, the cartridge engagement structure is designed to surround, in the assembled state, at least one of the cap and the neck portion of the cartridge body.

In this type of embodiment, the symmetry and tight tolerances of the neck portion of the cartridge body and/or the cap are exploited for the alignment.

The adapter engagement structure may especially surround, in the assembled state, the cap and at least a part of the neck portion. Here, the length of the normally "dead" neck portion is used for guiding.

In some embodiments, the adapter engagement structure has anti-rotation ribs, the anti-rotation ribs being designed to engage the adapter, thus preventing a relative rotation between the adapter and the alignment device in the engaged state. Thereby, it ensured that the adapter is displaced towards the cartridge in a pure translational motion, i.e. without superimposed rotation, which is favourable with respect to tightness. Favourably, the anti-rotation ribs extend along the common longitudinal axis in the engaged state.

Such anti-rotation ribs may also be present in a venting device as discussed above and further below.

In some embodiments, the alignment device is designed not to radially extend, in the assembled state, beyond the cartridge body. The term "radially" refers to a direction perpendicular to the cartridge axis. This type of embodiment has the property that the radial dimension in the assembled state is given by the cartridge body and not further increased by the alignment device. This is favourable since the radial dimension determines the diameter of a corresponding cartridge department of the infusion device and thus the device thickness, which is generally desired to b as slim as possible.

In some embodiments, the alignment device has a general tubular shape, extending along the common longitudinal axis. For such embodiments, the cartridge engagement structure is typically formed , fully or partly, by the inner surface of a proximal tube section. The adapter engagement structure is typically included in a distal tube section and may be designed in various ways as discussed further below.

For some tubular designs of an alignment device, the alignment device includes a proximal tubular member and a distal tubular member. The proximal tubular member forms the cartridge engagement structure. The distal tubular member forms or includes the adapter engagement structure. The proximal tubular member and the distal tubular member are displaceable with respect to each other in guided way along a common tube axis. In the assembled state, the common tube axis corresponds to the common longitudinal cartridge axis and longitudinal cannula axis. Such a a telescopic design is particularly compact.

In some embodiments of an alignment device, the alignment device includes a cartridge block, the cartridge block being designed to prevent further relative displacement between the alignment device and the cartridge upon the cartridge block hitting the cartridge. Such a cartridge block may also be present in a venting device as discussed above and further below.

In some tubular designs that include a cartridge block, the cartridge block includes at least one projection member, the at least projection member projecting radial into an inner volume of the tube. The projection member may, e.g., have a dent-shape or be designed as rim or protrusion.

Alternatively or additionally, a cartridge block of the alignment device may include a proximal front surface of the tube. Further displacement is prevented upon the front surface the cartridge body.

In some embodiments of the alignment device, the cartridge engagement structure and the adapter engagement structure overlap in axial direction. As will be discussed below in more detail in the context of exemplary embodiments, such an overlap is favourable with respect to compactness in the connected state.

In some embodiments, the adapter engagement structure includes at least one of a distal section of an outer circumferential surface of the alignment device, an inner circumferential surface of a distal section of the alignment device, and an alignment cavity, the alignment cavity being formed in a distal front surface of the alignment device.

In some embodiments of an alignment device, the alignment device further including a venting member, the venting member being made from a hydrophobic and gas-permeable material, the venting member having an environment coupling surface and an opposed cartridge coupling surface, thus enabling gas transfer and disabling liquid transfer across the venting member between the environment coupling surface and the cartridge coupling surface. This type of alignment device is designed such that, when coupled the cartridge, the cartridge having a movable wall,
- the cartridge coupling surface fluidic couples to a no-liquid contacting outer surface of the movable wall, and
- the environment coupling surface fluidic couples to the environment.

Such an embedment combines the advantages of a venting device and an alignment device in a common, compact unit.

According to a still further aspect, the present disclosure is directed towards a cartridge kit. The cartridge kit includes an alignment device and a liquid drug cartridge as discussed above, with the cartridge and the alignment device being coupled or designed to couple via the cartridge engagement structure of the alignment device.

According to a still further aspect, the present disclosure is directed towards an adapter kit. The adapter kit includes an alignment device and an adapter as discussed above, with the adapter and the alignment device being coupled or designed to couple via the adapter engagement structure of the alignment device.

Both a cartridge kit and an adapter kit are favourably supplied to a user in a disposable packing, either readily pre-assembled or in separate pieces.

According to a still further aspect, the present disclosure is direct towards a method for coupling a cartridge as discussed above with an adapter as discussed above via an alignment device as discussed above.

According to a still further aspect, the present disclosure is directed towards a liquid drug cartridge for use in an ambulatory infusion device. A cartridge according to this aspect of the present disclosure has a cartridge body, the cartridge body extending along a longitudinal cartridge axis. The cartridge further has a distal end section, the distal end section including an a self-sealing, piercable septum, the septum being perpendicular to the cartridge axis. The cartridge further has a number of alignment members, the alignment members being distributed about an outer circumference of the cartridge body, the alignment members extending along the longitudinal cartridge axis.

The alignment members of this type of embodiment fulfills the same purpose as the distal adapter engagement structure of an alignment device as discussed above. The alignment members are accordingly designed for axial aligned engagement with an adapter.

In some embodiments of an a cartridge including alignment members, the number of alignment members is three or four. While three alignment members that my be equally distributed around the cartridge body with an angle of 120° between the alignment members are sufficient for axial guiding, four equally distributed alignment members with an angle of 90° between them are considered preferable with respect to symmetry and injection moulding production processes.

While a separate alignment device as disclosed above is favourable for use in combination with already designed or existing cartridges, such as pen-injector type cartridges, the present cartridge design is considered favourable for newly designed cartridges.

### Exemplary embodiments

In the following, exemplary embodiments in accordance with the present general disclosure will be discussed in more detail with reference to the Figures.
Figure 1 shows a cartridge that is coupled to an adapter in a misaligned way (also discussed above).
Figure 2, 2b schematically show an embodiment of a venting device in accordance with the present disclosure.
Figure 3 schematically shows the venting device of Figure 2a, 2b in an assembled state, connected to a cartridge, an adapter and an ambulatory infusion device.
Figure 4a, Figure 4b schematically show a further embodiment of a venting device in accordance with the present disclosure.
Figure 5 schematically shows a still further embodiment of a venting device in accordance with the present disclosure, connected to a cartridge.
Figure 6 schematically shows an ambulatory infusion device with a cartridge and venting device of Figure 5.
Figure 7 schematically sows an alignment device in accordance with the present disclosure.
Figure 8 schematically shows the alignment device of Figure 7, coupled to an adapter and a cartridge.
Figure 9 schematically shows a further embodiment of an alignment device in accordance with the present disclosure.
Figure 10 schematically shows a still further embodiment of an alignment device in accordance with the present disclosure.
Figure 11a and 11b schematically show a liquid drug cartridge for use in an ambulatory infusion device in accordance with the present disclosure.

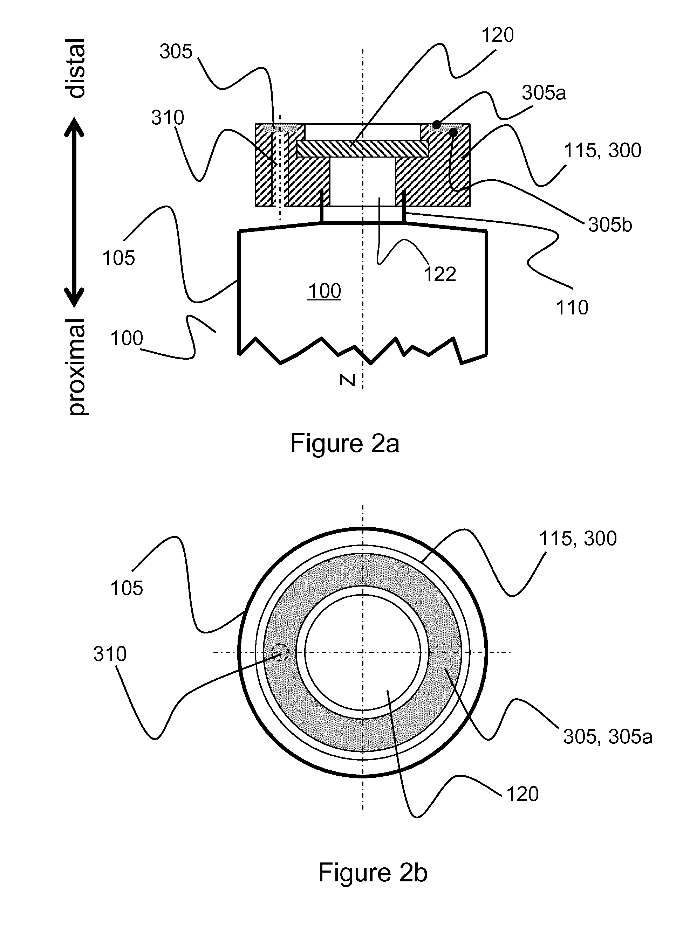

Figure 2a schematically shows a distal top section of a liquid drug cartridge 100 with a venting device 300 being included in a cap 115 of the cartridge 100 in a cross-sectional view, Figure 2b shows a corresponding top view. Cartridge 100 is exemplarily shown as having a cylindrical cartridge body 105 that is typically made from medical-grade glass or plastic. Cartridge body 105 has a constricted neck portion 110 (referenced in Figure 2a only). Cap 115 is typically made from metal or plastics and sealing attached to distal end of neck portion 110 via pressing or cramping. A self-sealing and piercable septum 120 is sealing arranged in cap 115. To this extent, cartridge 100 is a standard drug cartridge as it is typically used in pen-like injection devices (injection pens) and some ambulatory infusion devices, with the specific design being exemplary. For example, the cartridge body 105 may have a non-cylindrical cross section or may be formed by a fully or partly flexible pouch or bag. Instead of septum 120, the outlet may be realized differently, for example as male Luer coupler or any sort of suited proprietary fluidic coupler.

In accordance with the present disclosure, venting device 300 is included in cap 115, with the cap simultaneously serving as carrier member. The venting member is formed by a hydrophobic and gas-permeable membrane 305 that has the shape of a ring and is arranged in a concentric circumferential recess (not referenced) in a distal front surface of cap 105.

While environment coupling surface 305a is directly coupled to the environment, adjacent cartridge coupling surface 305b is coupled with a peripheral surface of cap 115 via elongated coupling channel 310 that is exemplarily show as bore which serves as coupling channel.

In Figures 2a, 2b, only a single bore 310 is shown as coupling channel, which, however is not essential. In dependence of the permeability of membrane 305, the inner device volume that needs to be vented, but also factors such as manufacturing aspects may lead to an arrangement with a different number of channels, e.g., two, three, four or six channels. The one or more coupling channels may also have a different cross section. For example, coupling channel 310 may have the shape of an elongated slot that extends about a section of the circumference.

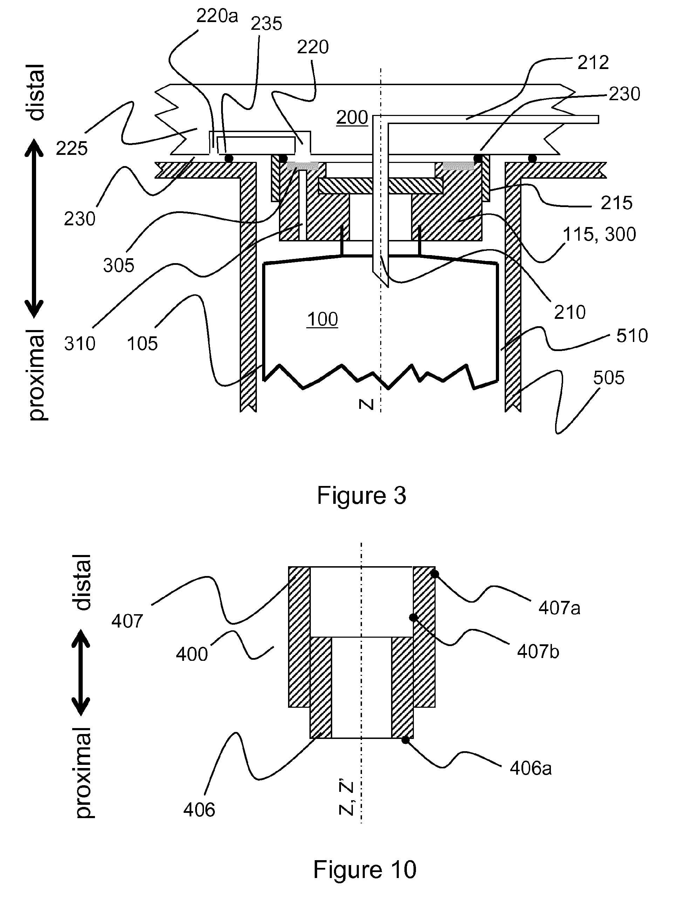

In the following, reference is additionally made to Figure 3. Figure 3 schematically shows cartridge 100 with venting device 300 when inserted into cartridge compartment 510 of an ambulatory infusion device, with element 505 indicating a housing of the ambulatory infusion device, thus defining cartridge compartment 510.

After insertion of cartridge 100, cartridge compartment 510 is at its distal end closed by adapter 200. Adapter 200 with adapter body 225 and guide 215 is coupled to housing 505 via an infusion device coupler, e.g. in form of a bayonet (not shown). An adapter cannula 210 extends, inside the adapter 200, into a drug channel 212 for coupling to an infusion cannula.

At the connection between adapter 200 and housing 505, adapter 200 includes two sealing elements that are exemplarily shown as O-rings 230, 235. Inner O-ring 230 is arranged such that it presses onto cap 115, thus axially supporting cartridge 100. Outer O-ring 235 presses onto housing 505. O-rings 230, 235 form a fluidic sealing that is tight with respect to both liquids, in particular insulin and water, and with respect to gas, in particular air.

In accordance with the present disclosure, adapter 200 includes a through-going venting channel 220. "Through-going" is meant in the sense that chat venting channel 220 does not include elements such as valves or membranes that would need to be passed by a gas flow through venting channel 220. One end (not referenced) of venting channel 220 is aligned with the environment coupling surface 305a (referenced in Figure 2). The other end 220a of venting channel 220 ends in a gap (not referenced) that exists between housing 505 and adapter body 225, thus bridging the O-ring sealings 230, 235. The gap is part of or is fluidic coupled to the environment.

As far as air is concerned, the inner volume of cartridge compartment 510 is accordingly coupled to the environment via coupling channel 310, membrane 305, and venting channel 220. With respect to water, however, the inner volume of cartridge compartment 510 is isolated from the environment because of the hydrophobic properties of membrane 305. Venting channel 220 may end at any other suited peripheral surface of adapter body 225. The end of venting channel 220 should be arranged such that free air flow is maintained but it is - as far as possible - protected against the intake of dirt or particles that may otherwise clog venting channel 220.

As can be seen in Figure 3, a gap (not referenced) is present in cartridge compartment 510 between the outer wall of cartridge body 105 and the surface of housing 505. Via this gap, the piston that is located in a (not shown) proximal section inside cartridge body 105, is also coupled with the environment with respect to air, thus venting the inner volume of cartridge compartment 510.

Besides venting cartridge compartment 510, Figure 3 shows an additional favorable property of a venting arrangement in accordance with the present disclosure. In some cases, septum 120 tends to leak. Besides drug delivery to the patient being interrupted or at least negatively effected in this case, many liquid drugs, such as typical liquid insulin formulations, are corrosive. At least a portion of the drive system of the ambulatory infusion device, in particular a plunger rod (not visible), typically projects into cartridge compartment 510 and may accordingly be damaged. In the shown arrangement, however, all connections between septum 120 (referenced in Figure 2) and the inner volume of cartridge compartment 510 are blocked for liquid by membrane 305 and O-ring sealings 230, 235. Even in case of a septum leakage, critical parts of the infusion device, in particular a plunger rod, do accordingly not come into contact with the drug.

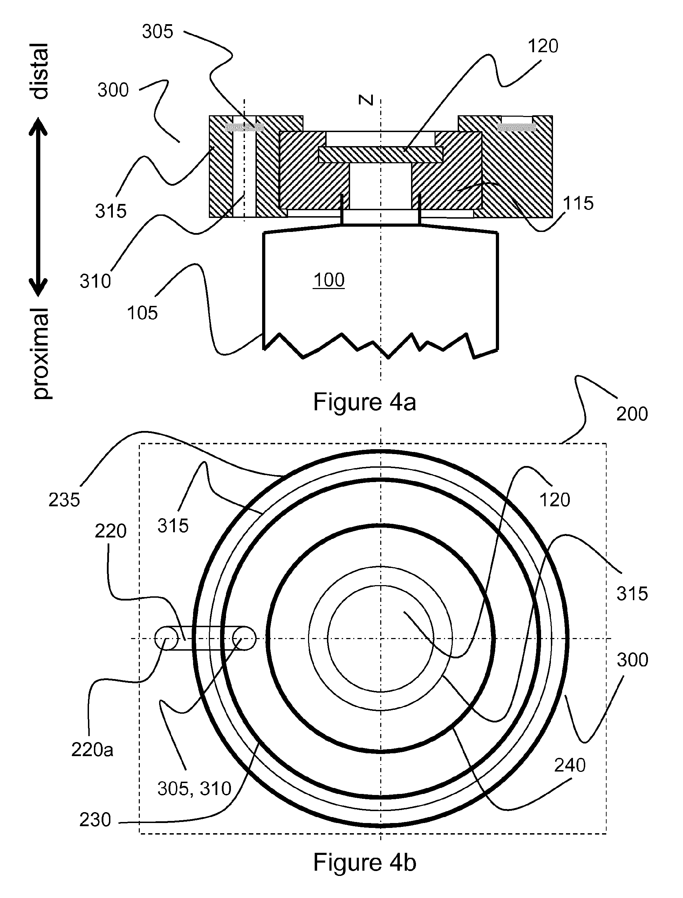

Figures 4a, 4b schematically illustrate a further embodiment of a venting device in accord accordance with the present disclosure. Figure 4a shows venting device 300 when assembled to cartridge body 105.

In contrast to the above-described embodiment, venting device 300 is not included in cap 115 of cartridge 100 (visible in Figure 4a only) and is accordingly not integral with cap 105. Instead, carrier member 315 is provided as dedicated component that is typically realized as injection-molded plastic component. Membrane 305 and coupling channel 310 are arranged in substantially the same way as they are in the above-described embodiment. Cartridge 100 and venting device 300, in combination, form a compact cartridge assembly.

The embodiment of Figure 4a has the particular property that it does not require a special or modified design of the cartridge itself. Cartridge 100 (visible in Figure 4a only) may, e.g., be a standard cartridge as typically used in pen-type injection devices. Carrier member 315 is favorably realized as cap that fits over cap 115 via snap fit or the like by a movement in proximal direction relative to cartridge 100. Once assembled, venting device 300 can favorably not be removed without damaging venting device 300 and/or cartridge 100.

Venting device 100 may alternatively assembled to cartridge 100 in other ways. Carrier member 315 may, e.g. be a ring with a slit, thus allowing carrier member 315 to be temporarily widened to be fit over cap 115 by radial movement.

Figure 4b schematically shows the geometric arrangement when the combination of cartridge 100 and venting device 300 is assembled with adapter 200 in top view.

While the general design of adapter 200 is similar to the arrangement shown in Figure 3, the arrangement of venting channel 220 and the O-ring sealings is slightly different. In this type of embodiment, three O-rings 230, 235, 240 or functionally equivalent sealing components are present in adapter 200. The arrangement of O-rings 230, 235 is equivalent to the description as given with reference to Figure 3. That is, O-ring 230 presses, in the assembled state, onto carrier member 315 in axial direction, while O-ring 235 presses onto the infusion device housing (not shown). Both O-rings are bridged by venting channel 220 with outlet 220a.

Additional innermost third O-ring 240 also presses onto carrier member 315 and is arranged between septum 120 and membrane 305, thus providing a fluidic separation between septum 120 and membrane 305. Alternatively, an arrangement with two O-rings may be used, with either of O-rings 230, 240 being omitted.

Like in the previously discussed embodiment, septum 120 is fluidic decoupled from the inner volume of the cartridge compartment 510 (see Figure 3) in the assembled state, thus preventing damage in case of a septum leakage.

For a cartridge with included venting device, as shown in Figures 2, 3, anti-rotation ribs may optionally be provided at the outer circumferential surface of the cap 115, the anti-rotation ribs extending in longitudinal direction. Those ribs engage guide 215 of the adapter 200, thus preventing relative rotation during assembly, which is favorably with respect to tightness as discussed above. In an analogue way, such anti-rotation ribs may be provided at the outer circumferential face of the carrier member 305 if the venting-member is provided separately.

A number of state-of-the art ambulatory infusion devises have a cartridge compartment 510 that is loaded as shown here, by inserting drug cartridge 100 in axial direction and closing the opening by disposable adapter 200. Alternatively, however, no disposable adapter may be present and cartridge compartment 510 ma be closed by a hinged door or the like that is part of device housing 505. Since, according to the present disclosure, venting member is part of the cartridge assembly, venting devices in accordance with the present disclosure may be used in combination with such devices as well.

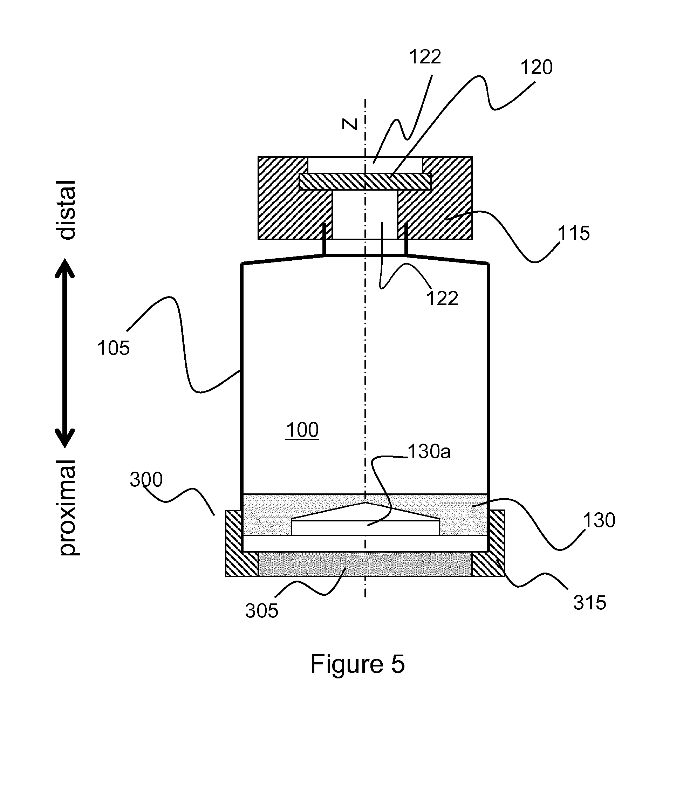

Figure 5 shows a further exemplary arrangement of a cartridge 100 in combination with a venting device in accordance with the present disclosure. Besides the integrated the venting device as discussed in the following, the design of cartridge 100 corresponds to the previously described embodiments. Therefore, the same reference numbers are used for identical or corresponding elements.

Cartridge 100 is generally designed in the same way as in the previously discussed embodiment, with Figure 5 additionally showing piston 130 that is sealing received inside cartridge body 105 and slidable along longitudinal cartridge axis Z from proximal towards distal for expelling drug.

The venting device is arranged at the proximal end cartridge body 105. Carrier member 315 of the venting device is designed as disk-shaped cap that tightly fits around the proximal end of cartridge body 105 is designed, e.g., as injection molded plastic component. Membrane 305 of the venting device is supported and fully surrounded by carrier member 315. While membrane 305 is shown with the same thickness as carrier member 315, this may not be the case in a specific embodiment. In particular, membrane 305 may be considerably thinner.

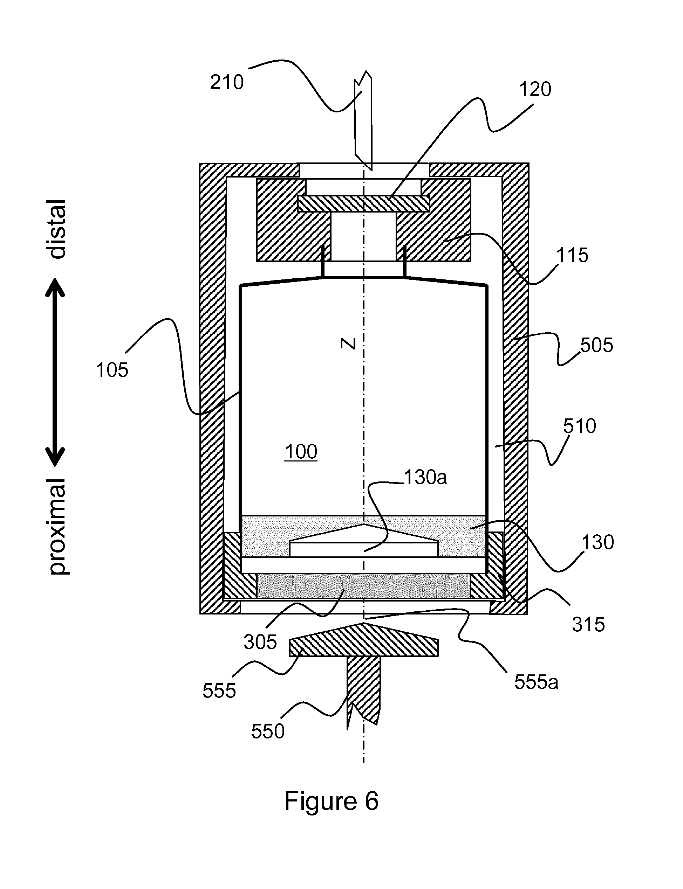

Figure 6 schematically shows cartridge assembly 100, 300 of Figure 5 when assembled into cartridge compartment 510 of an ambulatory infusion device, cartridge compartment 510 being formed by housing walls 505 as described above.

Figure 6 further shows cannula 210 for piercing septum 120, thus establishing fluidic access to the inner volume of cartridge 100. In an initialization phase prior to starting the infusion, piercing cannula 210 is moved in proximal direction to pierce the septum 120. Favorably, the ambulatory infusion device includes a corresponding cannula drive for this purpose, the cannula drive being controlled via control circuitry of the ambulatory infusion device. Suited arrangements are known in the art.

Figure 6 further shows plunger rod 550 of the ambulatory infusion device that is arranged proximal of drug cartridge 100 and carries, at its distal end, pusher plate 555. Plunger rod 550 with pusher plate 555 forms part of a typically electric drive system of the ambulatory infusion device.

Figure 6 shows plunger rod 550 in an initial, most proximal or fully retracted position. In the initialization phase, plunger rod 550 with pusher plate 555 is moved forward in distal direction. During this process, pointed distal end 555a of pusher plate 555 ruptures membrane 305 and proceeds its motion until pusher plate 555 is seated in the corresponding recess 130a of piston 130. Further advancing plunger rod 550 will result in piston 130 being pushed forward in distal direction, thus expelling liquid drug.

A number of variants and modifications are well possible. For example, the pusher plate 555 may have the shape of a cylindrical disc without pointed distal end 555a or may be designed for coupling to piston 130 in a different way, e.g. via a screw or snap fit. In some embodiments, recess 1 30a may not be present.

It can be seen that in the initial state as shown in Figure 6, both septum 120 and membrane 305 each form a sterile barrier, thus resulting all drug-contacting elements in a sterile state. In this configuration, the ambulatory infusion device may be stored for an extended time period up to several years, with cartridge 100 being readily filled with liquid drug and being assembled into the infusion device.

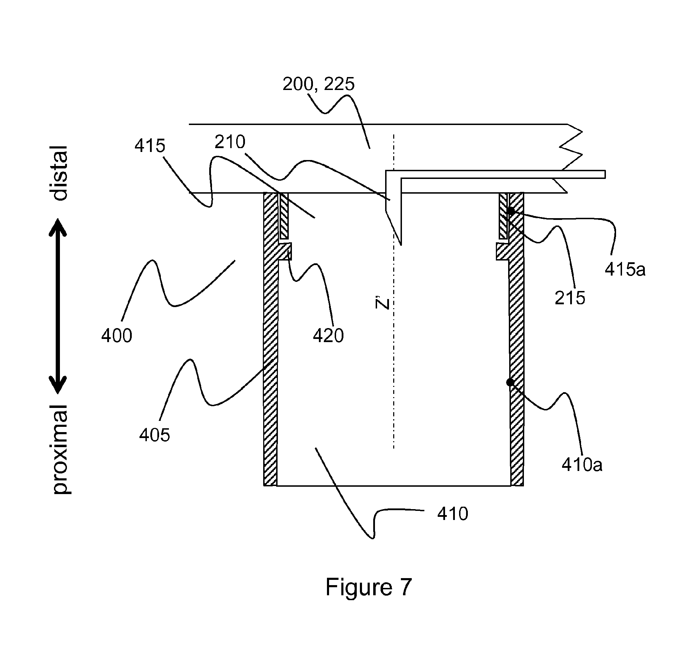

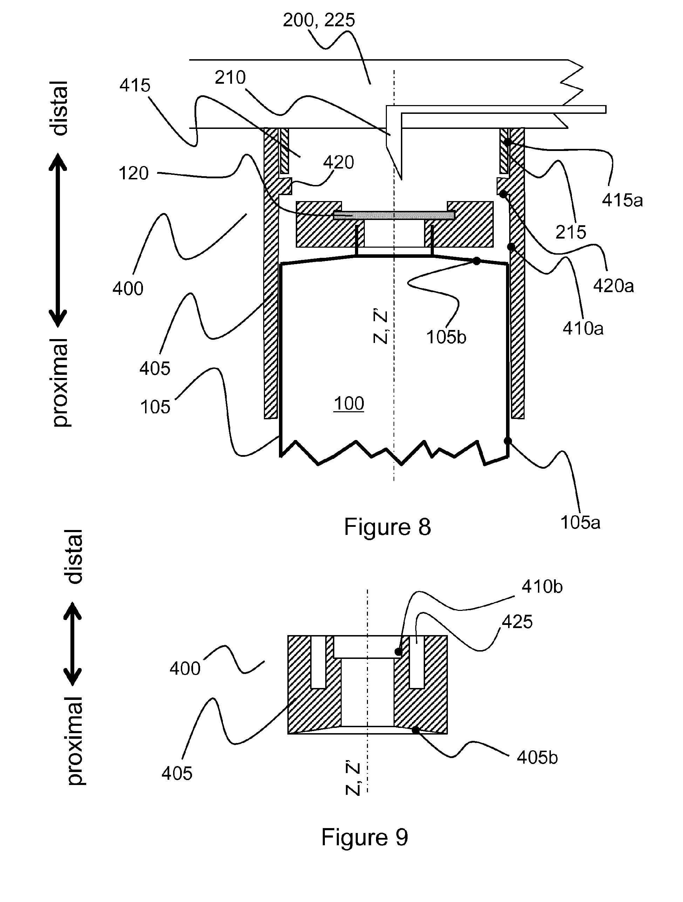

Figure 7 and Figure 8 illustrate an alignment device 400 in accordance with the present disclosure and its application. In the following description of alignment devices, adapters and cartridges that couple to the alignment device are generally assumed to be designed in the same way as discussed before in the context of venting devices, with identical or corresponding elements having the same reference numbers.

Figure 7 shows alignment device 400 when attached to adapter 200. The walls of guide 215 are parallel to longitudinal cannula axis Z' of adapter cannula 210 and perpendicularly project from an underside (not referenced) of adapter 200 in proximal direction. In the assembled state, guide 215 engages and couples with the distal adapter engagement structure of the alignment device.

Alignment device 400 has a general tubular shape with body 405. In a distal section, the inner tube diameter corresponds to the outer diameter of guide 215 such that it fits over guide 215 smoothly and with little play. The distal tube section, and in particular its inner surface 41 5a, accordingly forms the distal adapter engagement structure 415.

In a proximal section, the inner tube diameter corresponds to the outer diameter of a drug cartridge body such that it fits over the cartridge body smoothly and with little play. The proximal tube section, and in particular its inner surface 410a, accordingly forms cartridge engagement structure 410. Exemplarily, both diameters are identical in the embodiment of Figure 7. Between adapter engagement 415 and cartridge engagement structure 410, a ring-shaped protrusion 420 is arranged that projects into tubular body 405.

Figure 8 shows alignment device 400 readily assembled to adapter 200. Adapter 200 and alignment device 400 may be provided in this way, readily attached to each other. Alternatively, however, they are provided separately and assembled only prior to use, e.g. by a user, such as a patient.

Figure 8 shows the situation when coupling adapter 200 together with alignment device 400 to a drug cartridge 100. Adapter 200 is moved, together with attached alignment device 400, in proximal direction towards cartridge 100. During this process, circumferential surface 105a of cartridge body 105 is in guided, preferably smooth and substantially play-free sliding engagement with inner tubular surface 410a of cartridge engagement structure 410 (referenced in Figure 7 only), resulting in cartridge longitudinal axis Z and cannula longitudinal axis Z' being aligned. Cartridge engagement structure 410 does not need to extend over the total length of cartridge body 105 as long as it is sufficiently long to provide a guide and prevent tilting. Typically, it should have an axial overlap with cartridge body 105 in a range of some millimeters. To ensure proper alignment when adapter cannula 210 pierces septum 120, cartridge engagement structure 410, i.e. inner circumferential surface 410a of tubular body 405, must be sufficiently long to ensure that the sliding contact between surface 105a of cartridge body 105 and inner tubular surface 410a is established before the sharpened tip of adapter cannula 210 pierces septum 120. The assembly process is complete when proximal surface 420a of protrusion 420 hits distal end 105b of cartridge body 105, thus preventing further relative displacement. Protrusion 420 accordingly serves as cartridge block. Other kinds of projection members, such as bumps or dents, may serve as cartridge block, too.

In a variant of this embodiment, adapter engagement 41 5 may be realized by the outer circumferential surface of body 405 in a distal section of body 405 rather than the inner circumferential surface.

Cartridge 100 and adapter 200 may be assembled via alignment device 400 prior to inserting cartridge 100 - together with alignment device 400 - into the cartridge compartment of an infusion device. Alternatively, cartridge 100 may be inserted into the cartridge compartment prior to assembling it with adapter 200 via alignment device 400. In a further variant, cartridge 100 and alignment device 400 are assembled first and subsequently attached to adapter 200.

Figure 9 shows a further alternative embodiment of alignment device 400. Here, the adapter engagement structure is realized as a circumferential slot 425 in a distal front surface (not referenced) of device body 405, slot 425 being an alignment cavity. Slot 425 is coaxial with tubular body 405 and is designed for axial aligned engagement with the adapter, e.g. a guide 215 as shown in Figure 8, in a smooth sliding and preferably substantially play-free fit. The cartridge block is realized by proximal front surface 405b of body 405 that is shaped to fit to the distal front surface of the cartridge body. The hollow inner volume (not referenced) of device body 405 receives the neck of a cartridge body in a proximal section (not referenced) and the cap of the cartridge in a wider distal section. Proximal front surface 405b and inner circumferential surface 410b in the distal section, serve, in combination, for guided engagement with the cartridge body.

Alignment device 400 as shown in Figure 9 is favorable realized for a snap-fit engagement with the cartridge.

In comparison with the embodiment of Figure 7 and Figure 8, the embodiment of Figure 9 allows a slimmer design since alignment device 400 does not surround cartridge body 105. For this type of embodiment, the axial length of the constricted neck portion of the cartridge body is exploited for the alignment.

Figure 10 shows a further alternative embodiment of alignment device 400. Here, alignment device 400 is realized by proximal tubular member 406 and distal tubular member 407, the tubular members 406, 407 being arranged in a telescopic way and being displaceable with respect to each other along their common longitudinal axis with a guided and smooth fit. The adapter engagement structure is realized by outer circumferential surface 407a of distal tubular member 407. The inner volume of alignment device 400 receives the constricted neck portion of the cartridge body in the area of proximal tubular member 406 and the cap in the area of distal tubular member 407. Proximal front surface 406a of proximal tubular member 406 and inner circumferential surface 407b of distal tubular member 407, in combination serve in combination, for guided engagement with the cartridge body. Like in the embodiment of figure 10, the length of the constricted neck portion of the cartridge body is exploited for the alignment.

For assembly, alignment device 400 is first arranged between cartridge and adapter. Then, the adapter is displaced together with distal member 407 in proximal direction towards the cartridge and proximal tubular member 406 until the adapter cannula pierces the septum of the cartridge.

The various embodiments of an alignment device 400 as discussed above may further include anti-rotation ribs as described above in the context of venting devices.

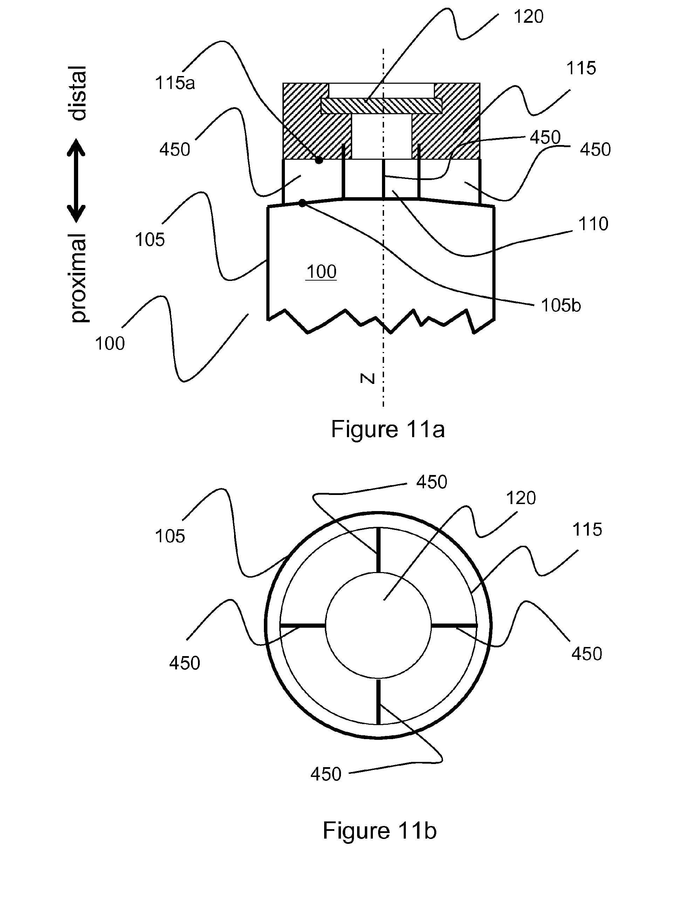

Figure 11a, 11b show a liquid drug cartridge for use in an ambulatory infusion system in accordance with the present disclosure. The overall design of cartridge 100 is similar to the previously described embodiments. However, cartridge 100 of this embodiment includes a number of alignment members 450 that are formed integral wit cartridge body 105, thus avoiding the need of a dedicated alignment device. Alignment members are realized by four wings 450 that are equally distributed about cartridge body 105 and extend along longitudinal cartridge axis Z between distal end 105b of cartridge body 105 and proximal front surface 115a of cap 115, with the radial dimension corresponding to the cap radius. Cartridge 100 is designed to couple with an adapter that may be generally designed as in the previously discussed embodiments, but includes a counter coupling structure that engages with wings 450, resulting in cartridge 100 being guided along the longitudinal cartridge axis Z in a substantially play-free way.

While various aspects of venting devices and of alignment devices are discussed above separately, they may well be combined in a common device. That is, a venting device as discussed above may be designed to fulfill at the same time the function of an alignment device. Vice versa, a venting member, in particular a hydrophobic and gas-permeable membrane, may integrated into an alignment device as discussed, with the body of the alignment device simultaneously serving as carrier member of the venting device.

## Claims

1. Venting device (300), including
a) a venting member (305), the venting member (305) being made from a hydrophobic and gas-permeable material, the venting member (305 )having an environment coupling surface (305a) and an opposed cartridge coupling surface (305b), thus enabling gas transfer and disabling liquid transfer across the venting member (305) between the environment coupling surface (305a) and the cartridge coupling surface (305b),
b) a carrier member (315), the carrier member (315) structurally supporting the venting member (305),
the carrier member (315) being part of or designed for assembly to a cartridge (100), the cartridge (100) storing a liquid drug in an inner volume and having a movable wall (130), the movable wall (130) decreasing the inner volume upon the stored liquid drug amount decreasing,
wherein the venting device (300) is designed such that
- the venting device (300) and the cartridge form, in the assembled state, a compact common cartridge assembly (100, 300), and, after inserting the cartridge assembly (100, 300) into a cartridge compartment of an ambulatory infusion device,
- the cartridge coupling surface (305b) fluidic couples, to a non liquid contacting outer surface of the movable wall, and
- the environment coupling surface (305a) at the same time fluidic couples to the environment.

2. Venting device (300) according to Claim 1, wherein the venting member (305) is a membrane, the membrane having lateral dimensions that are large as compared to a membrane thickness.

3. Venting device (300) according to either of the preceding claims, wherein the environment coupling surface (305a) and the cartridge coupling surface (305b) have a common normal axis, the normal axis being, in the assembled state, parallel to a longitudinal cartridge axis (Z).

4. Venting device (300) according to either of the preceding claims, wherein the carrier member (315) includes an elongated coupling channel (310), the coupling channel (310) fluidic coupling the environment coupling surface (305a) or the cartridge coupling surface (305b) with a peripheral surface of the venting device (300).

5. Venting device (300) according to either of the preceding claims, wherein the carrier member (315) surrounds, in the assembled state, at least a portion of a circumference of the cartridge (100).

6. Venting device (300) according to either of the preceding claims, wherein the carrier member (315) forms a cap, the cap the being designed to be put over a distal end section of the cartridge (100).

7. Venting device (300) according to either of the preceding claims,
- wherein the venting device is designed to couple to a liquid drug cartridge (100), the cartridge (100) having an elongated cartridge body (105) that extends along a longitudinal cartridge axis (Z) and has, at its distal end, a constricted neck portion (110), the cartridge (100) further including a cap (115) with a piercable septum (120) distal from the neck portion (110), the septum (120) being perpendicular to the cartridge axis (Z),
- wherein the venting device is further designed to couple to an adapter, the adapter (200) including an adapter cannula (210) with a longitudinal cannula axis (Z') to pierce the septum (120), and an infusion device coupler to mechanically couple to and engage with a housing of an ambulatory infusion device,
wherein the venting device (300) includes
- a proximal cartridge engagement structure (410), the cartridge engagement structure (410) being designed for axial aligned engagement with a distal end section of cartridge body (105),
- a distal adapter engagement structure (415), the adapter engagement structure (415) being designed for axial aligned engagement with the adapter (200),
thus enabling a coupling of the cartridge (100) with the adapter (200) via the venting device (300), wherein the adapter (200) and the cartridge (100) are, during the coupling, aligned by the cartridge engagement structure (410) and the adapter engagement structure (415), respectively relative to each other such that the longitudinal cartridge axis (Z) and the longitudinal cannula axis (Z') form a common longitudinal axis.

8. Venting device (300) according to either of the preceding claims, wherein the carrier member (315) is designed for assembly to the cartridge (100) via at least one of snap-fit, force fit, or cramping.

9. Cartridge assembly (100, 300) for use in an ambulatory infusion device, wherein the cartridge assembly (100, 300) includes a cartridge (100) and a venting device (300) according to either of claim 1 to claim 8.

10. Cartridge assembly (100, 300) according to claim 9, wherein the venting device (300) is arranged at a distal end section of the cartridge (100), the distal end section including an outlet for connecting to an infusion cannula.

11. Cartridge assembly according to claim 10, wherein the outlet includes a self-sealing, piercable septum (120).

12. Cartridge assembly (100, 300) according to either of claim 10 or claim 11, wherein the cartridge (100) further includes a piston (130) and an elongated cartridge body (105), the cartridge body (105) having a longitudinal cartridge axis (Z), the piston (130) being sealing arranged inside the cartridge body (105) and being displaceable along the longitudinal cartridge axis, the piston (130) having a liquid-contacting distal end and a non-liquid contacting proximal end, wherein the cartridge contact surface (305b) fluidic couples to the proximal end.

13. Cartridge assembly (100, 300) according to claim 12, wherein the venting device (300) is arranged at a proximal end section of the cartridge (100), such that the cartridge contact surface (305b) is adjacent to a proximal piston front surface and the venting device (300) tightly covers an open proximal end of the cartridge body (105).

14. Cartridge assembly (100, 300) according to claim 13, wherein the venting member (305) is designed to be ruptured by a plunger rod (550, 555) of an ambulatory infusion device.

15. Adapter (200) for coupling a cartridge assembly (100, 300) according to either of claim 9 to claim 14 with an infusion cannula and an ambulatory infusion device, the adapter (200) including
a) an infusion device coupler to mechanically couple to and engage with a housing of the ambulatory infusion device,
b) a drug channel (212) to fluidic couple the inner volume of the cartridge (100) with an infusion cannula, and
c) a venting channel (220) for establishing, in the assembled state, a fluidic tight coupling of the environment and the environment contacting surface (305a) and the environment via the venting channel (220).

16. Adapter-cartridge unit, the adapter-cartridge unit including a cartridge assembly (100, 300) according to either of claim 9 to claim 14 and an adapter (200) according to claim 15.

17. Ambulatory infusion device, the ambulatory infusion device including
a) a cartridge assembly (100, 300) according to claim 14,
b) a drive unit with a linearly displaceable plunger rod (550), the plunger rod being designed to rupture the venting member (305) and to subsequently engage the distal piston end,
c) a venting channel, the venting channel fluidic coupling the environment coupling surface (305a) with the environment.
